# EUROPÄISCHE PATENTANMELDUNG

(11) **EP 2 452 644 A2**
(43) Veröffentlichungstag der Anmeldung: **16.05.2012**
(21) Anmeldenummer: 11188352.6
(22) Anmeldetag: 09.11.2011
(51) Int. Cl.: A61B 17/88, A61B 17/17

(54) **Repositionszange mit einer Bohrhilfe**

(30) Priorität: 10.11.2010 DE 102010051919
(71) Anmelder: Karl Storz GmbH & Co. KG, 78532 Tuttlingen (DE)
(72) Erfinder: Berberich, Sascha, 78532 Tuttlingen (DE)
(74) Vertreter: Witte, Weller & Partner

(57) **Zusammenfassung**

Eine Repositionszange (10) dient zum Halten von zwei zu verbindenden Knochenteilen. Sie weist zwei Zangenhälften (12, 14) auf, die über ein Gelenk (16) scherenartig verbunden und in einer Zangenebene verschwenkbar sind. Jede Zangenhälfte (12, 14) weist zwei distalseitig des Gelenkes (16) ausgebogene Branchen (20, 22) und proximalseitig Griffteile (20, 22) auf. An einer Branche (22) ist eine Bohrhilfe (50) angebracht. Die Bohrhilfe (50) ist zumindest um eine Drehachse (58) drehbar, die senkrecht zur Zangenebene steht, und eine Führungsachse eines Führungskanals der Bohrhilfe (50) erstreckt sich in einer Drehstellung längs der Branche (22).

## Beschreibung

Die Erfindung betrifft eine Repositionszange zum Halten von zwei zu verbindenden Knochenteilen, mit zwei Zangenhälften, die über ein Gelenk scherenartig verbunden und in einer Zangenebene verschwenkbar sind, wobei jede Zangenhälfte distalseitig des Gelenkes eine seitlich ausgebogene Branche und proximalseitig ein Griffteil aufweist, und wobei an einer Branche eine Bohrhilfe bewegbar angebracht ist, die eine Führung für ein Bohrwerkzeug aufweist.

Eine Zange zum Klammern zweier Knochenteile ist aus der US 2,181,746 bekannt.

Zangen zum Repositionieren von zwei Knochenteilen finden insbesondere in der Unfallchirurgie Einsatz, bei der von einem Knochen abgesplitterte Knochenstücke wieder an dem Knochen angebracht werden sollen. Dies geschieht mittels Schrauben oder Fixierdrähten.

Um das abgesplitterte Knochenstück exakt an dem Knochen zu positionieren, finden Repositionszangen Einsatz.

Derartige Repositionszangen sind beispielsweise aus dem deutschen Gebrauchsmuster G 83 23 877.8 bekannt.

Aus der DE 10 2007 008 918 A1 ist eine Carpus-Zielzange mit integrierter axial-zentrischer Bohrführung nach Professor Krimmer bekannt. Eine der beiden Branchen zum Halten der Knochenteile weist eine Führung auf, durch die ein Kirschnerdraht oder ein Bohrstück, zum Beispiel ein Spiralbohrer, zielgenau in den Knochen eingeführt werden kann.

Aus der DE 32 03 451 A1 ist ein zangenartiges Instrument bekannt, das zum Halten von zwei Knochenteilen ausgebildet ist. In einer Branche sind am distalen Ende mehrerer Durchgangslöcher vorgesehen, durch die Bohrwerkzeuge in unterschiedlichen Ausrichtungen hindurchgeführt werden können.

Bei der eingangs genannten US 2,181,746 handelt es sich um eine Knochenklammer, die zwei Knochenstücke zusammenhält.

Seitlich steht von einer Branche eine relativ langerstreckte Bohrhilfe in Form einer Hülse ab. Mittig in der Hülse ist ein Führungskanal für ein Bohrwerkzeug vorhanden.

Die hülsenartige Bohrhilfe erstreckt sich in einer Grundstellung seitlich etwa unter einem rechten Winkel von der Branche weg, an der sie angebracht ist. Dabei erstreckt sich die Längsachse der Bohrführung in etwa in der Zangenebene.

In beiden Branchen sind längsverlaufende Längsschlitze ausgespart, so dass in der Grundstellung der Bohrhilfe durch die Längsschlitze der Branchen hindurch gebohrt werden kann.

Die eigentliche Bohrhülse sitzt auf einer kappenartigen Führung, so dass die Bohrhilfe, in Längsrichtung der Branche gesehen, auch seitlich verkippt werden kann. Dennoch muss das Bohrwerkzeug zumindest durch den Längsschlitz in der Branche hindurchgeführt werden, an der das Bohrwerkzeug montiert ist.

Nachteilig an dieser Vorrichtung ist, dass die Bohrhilfe seitlich zu weit abstehend ist und somit sehr sperrig baut.

Darüber hinaus sind die Freiheitsgrade der Ausrichtung des Bohrwerkzeuges dadurch eingeschränkt, dass immer durch die mittige zentrale Öffnung der Branche hindurch gebohrt werden muss, an der die Bohrhilfe montiert ist.

Dies schränkt den Operateur stark in seiner Freiheit ein, an welcher Stelle er die Bohrung ansetzen möchte. Bei Knochenklammern, die zwei Knochenteile bei einer Osteotomie halten sollen, ist das relativ unkritisch, denn die beiden zu verbindenden Knochenteile wurden zuvor mit genau definierten Trennstellen getrennt, um beispielsweise Fehlstellungen oder Überlängen zu beseitigen. Daher sind diese Knochenteile gut über die definierte Trennstelle zu fixieren.

Bei kompliziert geformten Knochensplittern benötigt der Operateur hohe Freiheitsgrade für die Bohrhilfe, um die Bohrkanäle auf eine Stelle richten zu können, die eine ausreichende Knochensubstanz für die Fixierung aufweist. Wenn das subkutan durchgeführt werden soll, muss diese Freiheit besonders vorhanden sein.

Es ist daher Aufgabe der vorliegenden Erfindung, eine Repositionszange zu schaffen, die wenig ausladend baut sowie einfach handhabbar ist, und die dem Operateur ein hohes Maß an Freiheit zum Setzen der Bohrung an den zu verbindenden Knochenteilen lässt.

Erfindungsgemäß wird die Aufgabe dadurch gelöst, dass die Bohrhilfe zumindest um eine Drehachse drehbar ist, die senkrecht zur Zangenebene steht, und wobei eine Führungsachse der Bohrhilfe in einer Drehstellung sich längs der Branche erstreckt.

Die Branchen werden zum Halten der Knochenteile auf diese zu bewegt und positionieren die beiden zu verbindenden Knochenteile zwischen den distalen Enden der Branchen. Die Ausrichtung der Führungsachse der Bohrhilfe in einer Drehstellung längs der Branche erlaubt es nun, gezielt in Richtung der zwischen den distalen Enden gehaltenen Knochenstücke zu bohren. Dieser Vorgang kann auch subkutan stattfinden, denn der Arzt weiß ja, dass die zu verbindenden Knochenstücke zwischen den Zangenspitzen gehalten sind. Eine Verschwenkung der Bohrhilfe um eine Achse senkrecht zur Schwenkebene erlaubt es, die Bohrhilfe nach wie vor an eine Branche, aber etwas im Abstand zur Zangenebene, zu bringen, so dass in allen Drehstellungen der Bohrhilfe jeweils die Knochenteile angezielt werden und nicht eine Branche getroffen wird, so dass keine Vorkehrungen getroffen werden müssen, damit man durch diese Branche hindurch bohren kann.

Außerdem ist es möglich, die ganze Bohrhilfe sehr kompakt auszubilden, da keine von den Branchen rechtwinklig abstehenden Führungskanäle vorhanden sind.

Dies erleichtert erheblich die Handhabung der Bohrhilfe und lässt dem Operateur zahlreiche Freiheitsgrade zur Ausrichtung der Bohrhilfe.

In einer weiteren Ausgestaltung der Erfindung ist die Bohrhilfe als Block ausgebildet, durch den sich die Führung als zumindest ein Führungskanal hindurch erstreckt.

Diese Maßnahme hat den handhabungsmäßigen Vorteil, dass eine sehr kompakte Bohrhilfe geschaffen werden kann, die von dem Operateur sehr einfach ergriffen werden und in die gewünschte Bohrrichtung ausgerichtet werden kann. Dazu braucht er nur den Block zu ergreifen und entsprechend um die Drehachse zu verdrehen, damit er die gewünschte Ausrichtung erhält.

In einer weiteren Ausgestaltung der Erfindung sind im Block zumindest zwei Führungskanäle ausgebildet.

Diese Maßnahme hat den Vorteil, dass mit einer Ausrichtung der Bohrhilfe gleich zumindest zwei Bohrkanäle bewerkstelligt werden können, indem nämlich durch die zumindest zwei oder mehr Führungskanäle mehrere Bohrwerkzeuge durchgeführt werden können oder dasselbe Bohrwerkzeug nach und nach durchgeführt werden kann, um mehrere Bohrkanäle für Fixierschrauben oder Fixierdrähte zu bewerkstelligen.

In einer weiteren Ausgestaltung der Erfindung verlaufen die Führungsachsen der mehreren Führungskanäle auf verschiedenen Höhenniveaus und/oder nicht-parallel zueinander, dennoch sind sie auf einen Bereich zwischen den beiden Branchen ausgerichtet.

Durch diese Ausgestaltung können unabhängig voneinander zwei für die Fixierung günstig und unterschiedlich ausgerichtete Bohrkanäle geschaffen werden, wobei sichergestellt ist, dass sich auch bei nicht-paralleler Ausrichtung die Bohrkanäle nicht treffen.

Dies erlaubt es dem Operateur, bei einer Ausrichtung der Bohrhilfe zwei Bohrkanäle nach und nach zu setzen, die eine optimale Ausrichtung zum Setzen der Fixierschrauben oder Fixierdrähte erlauben, so dass dieser Vorgang sehr rasch durchgeführt werden kann. Je nach Drehstellung und Ausrichtung können die Bohrkanäle sich kreuzen, jedoch ohne einander zu treffen, oder divergieren.

In einer weiteren Ausgestaltung ist der Block über einen sich in der Schwenkachse erstreckenden Bolzen mit der Branche verbunden.

Diese Maßnahme hat den Vorteil, dass das kompakte Bauteil der Bohrhilfe durch die Ausbildung als Block über einen einzigen Bolzen mit der Branche verbunden werden kann. Dadurch sind wenige Bauteile zur Verbindung zwischen Bohrhilfe und Branche notwendig, die auch wenige Bakteriennischen aufweisen, so dass eine solche Vorrichtung auch einfach gereinigt und desinfiziert werden kann.

In einer weiteren Ausgestaltung der Erfindung ist das Ausmaß der Drehbewegung um die Drehachse begrenzt.

Die zuvor erwähnte einfache Verbindung zwischen Branche und dem Block über einen Bolzen erlaubt nun grundsätzlich, den Block um 360° um die Drehachse zu verschwenken. Dies kann dann erwünscht oder günstig sein, wenn man den Block so einsetzen will, dass man das Bohrwerkzeug von beiden entgegengesetzten Richtungen durch den Block hindurch schieben möchte, insbesondere wenn die Führungskanäle nicht-parallel oder auf verschiedenen Höhen laufen. So kann man beispielsweise in einer Ausrichtung des Blockes aufeinander zu gerichtete Bohrkanäle setzen und in der um 180° verdrehten Stellung dann durch diese Bohrkanäle entsprechend divergierende Kanäle setzen.

Sind aber die Kanäle, wie zuvor erwähnt, schon in variablen Ausrichtungen in der Bohrhilfe ausgespart, kann es ausreichend sein, nur ein gewisses Maß der Drehbarkeit, beispielsweise um ein bestimmtes Winkelmaß nach links und nach rechts um den Bolzen vorzusehen.

In einer weiteren Ausgestaltung dazu sind am Block beidseits der Branche Anschläge vorgesehen, die eine Drehbewegung um die Drehachse begrenzen.

Diese Anschläge schaffen durch konstruktiv einfache Maßnahmen die zuvor erwähnte Begrenzung der Drehbarkeit.

Solche Anschläge können als Vorsprünge oder vorstehende Stifte oder dergleichen ausgebildet sein und somit als einfach zu bewerkstelligende und unkomplizierte Bauteile ausgestaltet werden. Dies erleichtert nicht nur die einfache Herstellung und die Handhabung, sondern auch die Reinigung und Desinfizierung einer solchen Repositionszange.

In einer weiteren Ausgestaltung der Erfindung ist die Bohrhilfe zusätzlich um eine Längsachse der Branche, an der diese angebracht ist, drehbar ausgebildet.

Diese Maßnahme hat den Vorteil, dass durch die Drehung um die Längsachse dem Arzt weitere Freiheitsgrade gegeben werden, um Bohrungen zu setzen.

Die Bohrhilfe ist ja etwas seitlich versetzt an der Branche angebracht. Wird die Zange dazu eingesetzt, dass ein von einem Knochen abgesplittertes Knochenstück wieder angebracht werden soll, können die Branchen so angesetzt werden, dass diese das abgebrochene Knochenstück etwa mittig erfassen. In einer Stellung der Bohrhilfe können dann eine oder mehrere Bohrungen auf dieser Seite der Branche gesetzt werden. Anschließend kann die Bohrhilfe beispielsweise um 180° um die Längsachse der Branche gedreht werden, so dass dann an einer diametral gegenüberliegenden Stelle das Knochenstück mit entsprechenden Bohrungen versehen werden kann.

Bei relativ großflächigen Knochenstücken können nun Bohrungen in mehreren unterschiedlichen Drehstellungen um die Längsachse der Branche gesetzt werden.

Zur Lagerung oder zum Transport kann dann die Bohrhilfe in eine Stellung gedreht werden, in der sie bei geschlossener Zange innerhalb der beiden Branchen zum Liegen kommt, was für ein Verstauen, Verpacken und Versenden günstig ist.

In einer weiteren Ausgestaltung der Erfindung ist die Bohrhilfe längs der Branche, an der diese angebracht ist, verschiebbar ausgebildet.

Diese an sich aus der eingangs erwähnten US 2,181,746 bekannte Maßnahme erlaubt nun noch eine zusätzliche Feinjustierung dadurch, dass die Bohrhilfe zusätzlich noch den Freiheitsgrad der Längsverschiebung längs der Branche aufweisen kann.

In einer weiteren Ausgestaltung der Erfindung sind die beiden Branchen der Zangenhälften distalseitig als nadelartige Dorne ausgebildet.

Diese Maßnahme hat den Vorteil, dass die Repositionierung subkutan durchgeführt werden kann, beispielsweise die Dorne durch die Haut hindurch gestochen werden können, um die darunter liegenden Knochenstücke zu repositionieren. Dies erleichtert den Operationsvorgang dahingehend, dass die Operationsstelle nicht vollständig geöffnet werden muss.

In einer weiteren Ausgestaltung der Erfindung weisen die Branchen, von der Mittellängsachse der Repositionszange aus gesehen, seitlich ausgebogene Abschnitte auf, deren nadelartige Dorne bei geschlossener Repositionszange aufeinander zu gerichtet sind.

Diese Maßnahme hat den Vorteil, dass die beiden zu verbindenden Knochenstücke punktuell zwischen den nadelartigen Dornen gehalten sind, wobei wegen der ausgebogenen Abschnitte ausreichend Platz um diese Stelle vorhanden ist, an der Bohrhilfe die entsprechenden Manipulationen vorzunehmen.

Dies wirkt sich insbesondere dann sehr vorteilhaft aus, wenn ein relativ kleines abgesplittertes Knochenstück an einem Knochen befestigt werden soll.

In einer weiteren Ausgestaltung der Erfindung ist die Bohrhilfe in der Nähe eines nadelartigen Dornes angeordnet.

Diese Maßnahme hat den Vorteil, dass durch die Nähe der Operateur ein sehr gutes Gefühl hat, wie die Bohrung, die ja durch die Bohrhilfe bewerkstelligt wird, ausgerichtet ist. Der Operateur sieht die Ausrichtung der Dorne, selbst wenn diese durch Gewebe durchgestochen sind, und er weiß dann, wie er die Bohrhilfe auszurichten hat, damit die Bohrung exakt die zu verbindenden Knochenstücke trifft, selbst wenn er die Knochenstücke nicht unmittelbar visuell erkennen kann.

Dies erleichtert die Handhabung erheblich.

In einer weiteren Ausgestaltung der Erfindung ist zwischen den Griffteilen der Zangenhälften eine Haltevorrichtung vorhanden, die die Griffteile und somit auch die Branchen in bestimmten Schwenkstellungen hält.

Diese an sich bekannte Maßnahme hat den Vorteil, dass in Kombination mit der erfindungsgemäßen Bohrhilfe der Repositioniervorgang besonders einfach und sicher durchführbar ist. Zunächst können mittels Verschwenken der beiden Zangenhälften die beiden zu verbindenden Knochenstücke in eine gewünschte Position zueinander gebracht werden, wobei dann die Haltevorrichtung die Zangenhälften in diesen Stellungen hält. Der Arzt kann sich dann voll auf die Ausrichtung der Bohrhilfe konzentrieren und die Bohrkanäle für die Fixierschrauben bzw. Fixierdrähte setzen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine Draufsicht auf ein erstes Ausführungsbeispiel einer Repositions-zange mit einer Bohrhilfe, die nur um einen Freiheitsgrad bewegbar ausgebildet ist,
- Fig. 2: eine abschnittsweise stark vergrößerte Draufsicht auf die Zange von Fig. 1, und zwar von der gegenüberliegenden Seite,
- Fig. 3A bis 3C: einen stark vergrößerten Ausschnitt der in Fig. 2 auf der linken Seite dargestellten Branche, mit drei verschiedenen Drehstellungen der Bohrhilfe, nämlich einer mittigen, einer in einer Richtung maximal verdrehten und der in die entgegengesetzte Richtung maximal verdrehten Drehstellung, wobei diese durch entsprechende Anschläge begrenzt werden,
- Fig. 4: eine stirnseitige Ansicht der in Fig. 1 auf der rechten Seite dargestellten Branche mit der Bohrhilfe, und zwar exakt auf die Spitze des Dornes zu gesehen,
- Fig. 5: eine entsprechende Ansicht von der gegenüberliegenden Seite, also von hinten bzw. von proximal nach distal,
- Fig. 6: eine stark vergrößerte, ausschnittsweise Darstellung eines praktischen Einsatzes der Repositionszange von Fig. 1,
- Fig. 7a: ein zweites Ausführungsbeispiel einer Repositionszange mit Bohrhilfe, wobei diese Bohrhilfe zusätzlich um die Längsachse der Branche, an der sie angebracht ist, schwenkbar ist,
- Fig. 7b: eine der Darstellung von Fig. 7a vergleichbare Stellung, wobei die Bohrhilfe in der Darstellung entgegen dem Uhrzeigersinn um die Branche verschwenkt worden ist,
- Fig. 7c: eine der Fig. 7a vergleichbare Stellung, wobei die Bohrhilfe um 180° im Uhrzeigersinn um die Längsachse der Branche verdreht worden ist,
- Fig. 7d: eine der Fig. 7a vergleichbare Drehstellung, wobei dargestellt ist, dass auch diese Bohrhilfe um eine Drehachse senkrecht zur Zangenebene verdrehbar ist,
- Fig. 8: eine Seitenansicht der Bohrhilfe von Fig. 7a mit auf die Zangenebene geneigter Führungsachse der Bohrung, und
- Fig. 9: eine der Fig. 8 vergleichbare Darstellung eines dritten Ausführungs-beispiels, bei dem die Bohrhilfe zusätzlich noch längs der Branche, an der sie angebracht ist, verschiebbar ausgebildet ist.

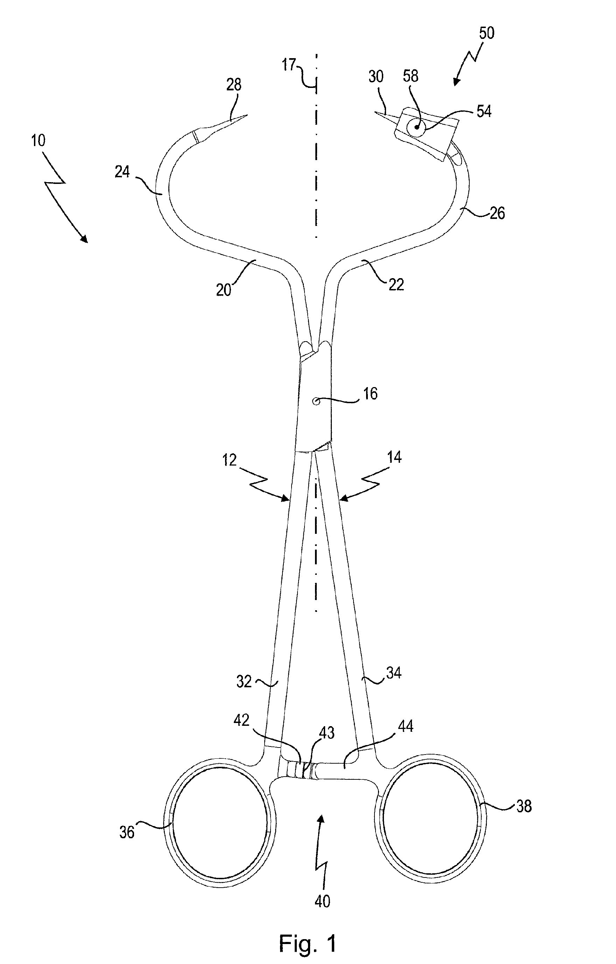

Ein in den Fig. 1 bis 6 dargestelltes erstes Ausführungsbeispiel einer erfindungsgemäßen Repositionszange ist in seiner Gesamtheit mit der Bezugsziffer 10 bezeichnet.

Die Repositionszange 10 weist zwei Zangenhälften 12 und 14 auf, die über ein Gelenk 16 scherenartig um dieses Gelenk 16 verschwenkbar miteinander verbunden sind. Aus Fig. 1 ist zu erkennen, dass die Zangenhälften 12 und 14, über eine Mittellängsachse 17 der Repositionszange 10 gesehen, etwa spiegelbildlich ausgebildet sind.

Die beiden Zangenhälften 12 und 14 können somit in einer Zangenebene 18, die in Fig. 1 in etwa der Zeichenebene entspricht, verschwenkt werden.

Distalseitig des Gelenkes 16 weist jede Zangenhälfte 12 bzw. 14 eine Branche 20 bzw. 22 auf.

Jede der Branchen 20 bzw. 22 weist einen seitlich sich in der Zangenebene 18 erstreckenden, gebogenen Abschnitt 24 bzw. 26 auf.

Die beiden gebogenen Abschnitte 24 bzw. 26 gehen jeweils distalseitig in einen etwa geradlinig ausgebildeten nadelartigen Dorn 28 bzw. 30 über, deren Spitzen aufeinander zu gerichtet sind und sich auf Höhe der Mittellängsachse 17 treffen können.

Proximalseitig des Gelenkes 16 weist jede Zangenhälfte 12 bzw. 14 ein Griffteil 32 bzw. 34 auf, das am proximalen Ende jeweils mit einer Fingeröse 36 bzw. 38 versehen ist.

In diesem Bereich erstreckt sich zwischen den Griffteilen 32 und 34 eine Haltevorrichtung 40, die aus zwei aufeinander zu gerichteten und sich überlappenden Rastleisten 42 bzw. 44 besteht, die jeweils von der Innenseite des Griffteiles 32 bzw. 34 nach innen gerichtet vorspringen.

In der Darstellung von Fig. 1 sind Zähne 43 der Rastleiste 42 zu erkennen, die mit entsprechenden, hier nicht ersichtlichen Zähnen der Rastleiste 44 zusammenwirken.

Mit anderen Worten sorgt die Haltevorrichtung 40 dafür, dass die Repositionszange 10 beispielsweise in der in Fig. 1 dargestellten Position verbleibt, wenn sie nicht bewegt wird.

Die Rastwirkung ist allerdings so, dass diese durch die Kraft einer menschlichen Hand, die die Repositionszange 10 ergreift, überwunden werden kann, so dass durch entsprechende Bewegung die Repositionszange 10 aus der Darstellung von Fig. 1 weiter geöffnet oder auch entsprechend geschlossen werden kann.

An der Branche 22 ist eine in ihrer Gesamtheit mit der Bezugsziffer 50 versehene Bohrhilfe montiert.

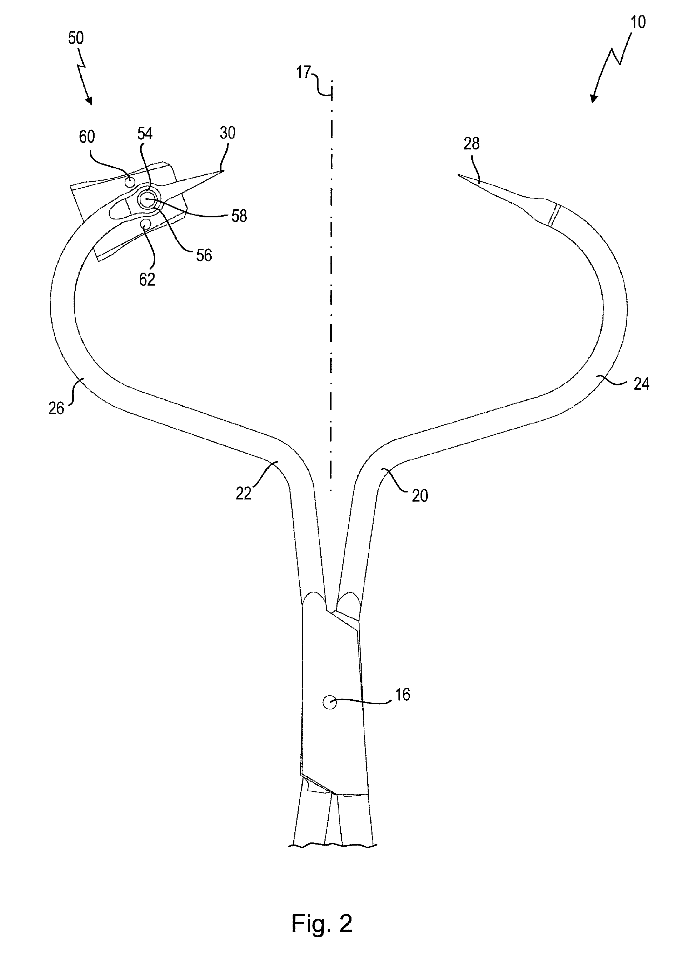

Wie insbesondere aus den Fig. 2 bis 5 näher ersichtlich ist, besteht die Bohrhilfe 50 aus einem etwa quaderförmigen Block 52.

Durch den Block 52 hindurch erstreckt sich ein Bolzen 54, der in einer Öse 56 in der Branche 22 montiert ist.

Die Bohrhilfe 50 kann um den Bolzen 54 gedreht werden, wobei die Mittellängsachse des Bolzens 54 eine Drehachse 58 der Bohrhilfe 50 darstellt.

Die Bohrhilfe 50 bzw. der Block 52 ist dadurch seitlich an der Branche 22 über den Bolzen 54 montiert, so dass die Bohrhilfe 50 grundsätzlich um 360° um die Drehachse 58 drehbar ist.

Im dargestellten Ausführungsbeispiel ist aber diese Drehbewegung um die Drehachse 58 dadurch begrenzt, dass am Block 52 zwei Anschläge 60 und 62 vorgesehen sind.

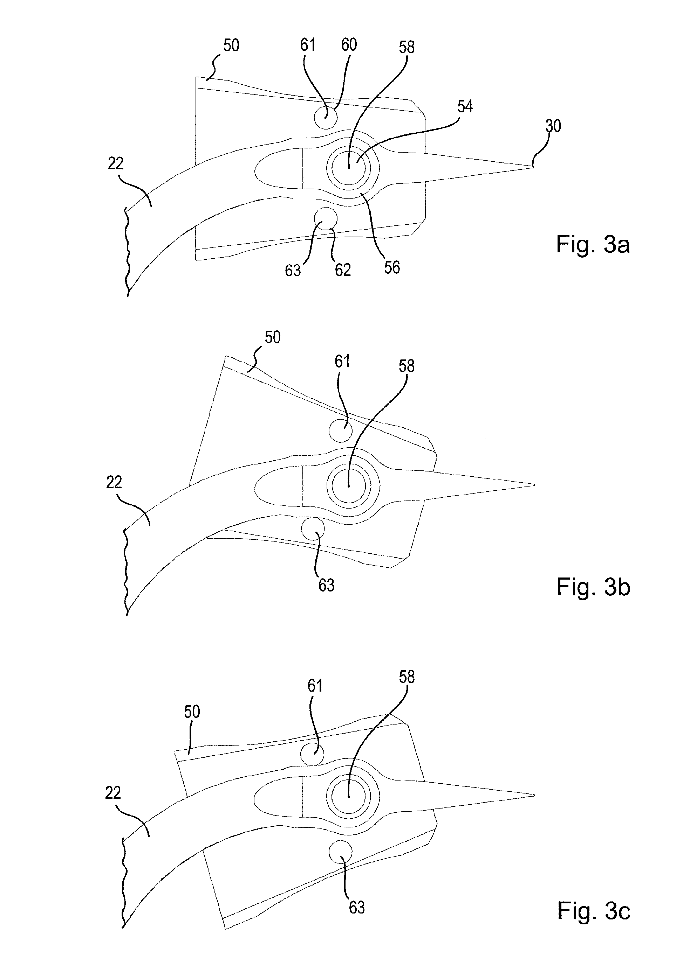

Wie insbesondere aus den Fig. 3A bis 3C sowie 4 und 5 ersichtlich, sind die Anschläge 60 bzw. 62 als von einer ebenen Außenseite des Blockes 52 in Richtung der Branche 22 vorstehende Zapfen 61 und 63 ausgebildet. Dabei erstreckt sich der Zapfen 61, wenn man längs der Branche 22 von proximal nach distal blickt, in der Darstellung der Fig. 3 links von der Branche 22, und der Zapfen 63 erstreckt sich rechts von der Branche 22.

Die Anordnung und der Abstand der Anschläge 60 bzw. 62 sind nun so, dass von einer "Mittenstellung", wie sie in Fig. 1, 2 und 3A dargestellt ist, die Bohrhilfe 50 um die Drehachse 58 im Uhrzeigersinn oder entgegen dem Uhrzeigersinn jeweils so lange verschwenkt werden kann, bis einer der Anschläge 60 bzw. 62 an der Außenseite der Branche 22 anstößt.

In dem dargestellten Ausführungsbeispiel beträgt der Schwenkbereich aus der Mittenstellung von Fig. 3A jeweils etwa 20°.

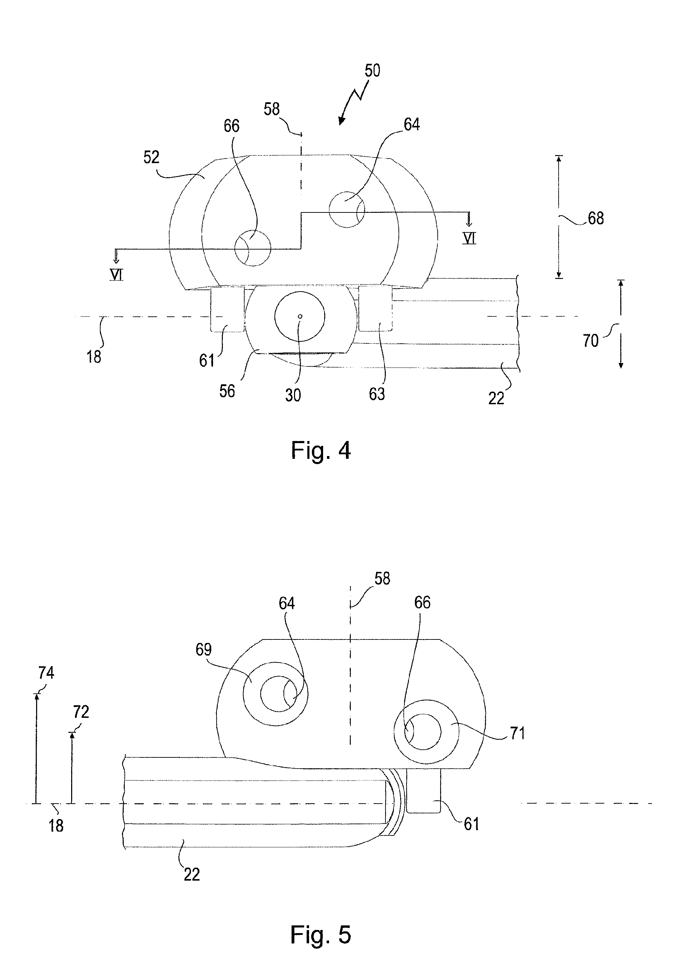

Aus den Darstellungen von Fig. 4 bis 6 ist zu erkennen, dass im Block 52 der Bohrhilfe 50 zwei Führungskanäle 64 und 66 vorhanden sind. Dabei ist ersichtlich, dass sich die beiden Führungskanäle 64 und 66 in einer als "Längsrichtung" bezeichneten Richtung komplett durch den Block 52 hindurch erstrecken. Es ist zu erkennen, dass die Führungskanäle 64 und 66 auf unterschiedlichen Höhenniveaus ausgebildet sind, der "obere" Führungskanal 64 auf dem Höhenniveau 74 und der "untere" Führungskanal 66 auf dem Höhenniveau 72.

Die Führungsachsen 65 bzw. 67 der Führungskanäle 64 bzw. 66 verlaufen etwa parallel zur Zangenebene 18, allerdings auf verschiedenen Höhenniveaus, sind jedoch - wenn man von proximal nach distal blickt, also in der Darstellung von Fig. 5 - aufeinander zu gerichtet bzw. laufen, in Richtung der Längserstreckung der Branche 22 an dieser Stelle gesehen, aufeinander zu.

Aus der Darstellung von Fig. 5 ist auch zu erkennen, dass die Führungskanäle 64 und 66 mit entsprechenden Einlaufschrägen 69 bzw. 71 versehen sind, um das Einbringen eines Bohrwerkzeuges, beispielsweise eines Spickdrahtes oder eines Drillbohrers, zu erleichtern.

Aus der Darstellung von Fig. 5 ist ferner zu erkennen, dass die Bauhöhe 68 des Blockes 52 nur unwesentlich größer ist als der Außendurchmesser 70 der Branche 22, das heißt der Block 52 baut höhenmäßig sehr kompakt. Ferner ist zu erkennen, dass die Branche 22 in dem Abschnitt, an dem der Block 52 montiert ist, abgeflacht ausgebildet ist. Dadurch ist die Bauhöhe in diesem Abschnitt gering gehalten.

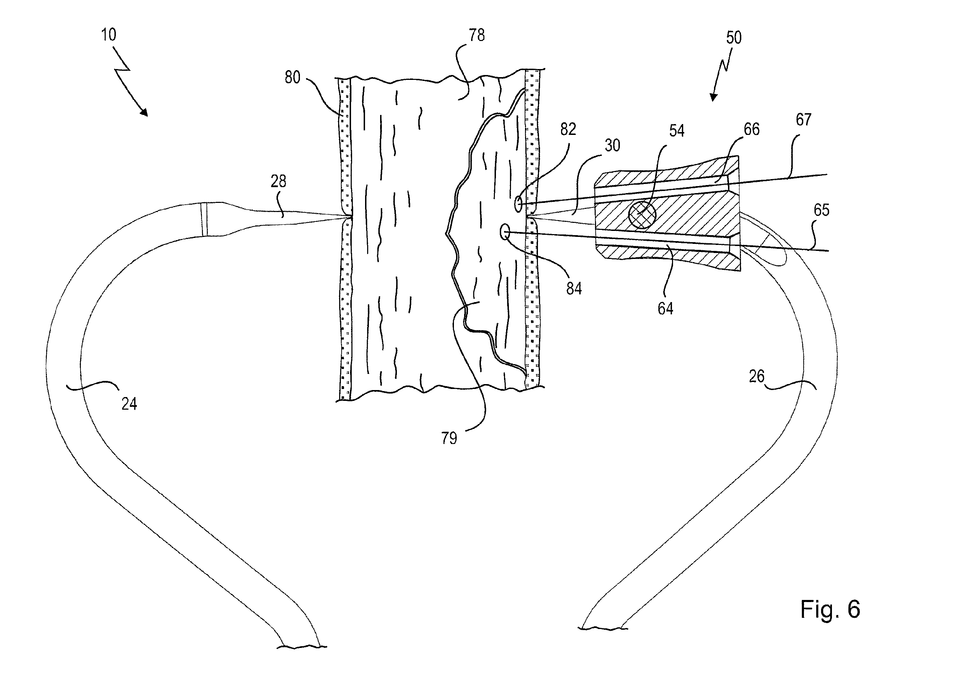

In Fig. 6 ist eine Handhabung der Repositionszange 10 dargestellt, wobei sie hier dazu dient, ein von einem Knochen 78 abgebrochenes Knochenstück 79 wieder an diesem zu fixieren.

In Fig. 6 ist zu erkennen, dass die Repositionierung dadurch geschehen kann, dass die nadelartigen Dorne 28 und 30 von der Außenseite durch die den Knochen 78 umgebende Haut 80 hindurchgestochen werden können, um das abgebrochene Knochenstück 79 lagegerecht an dem Knochen 78 zu positionieren.

In dieser Stellung, die durch die zuvor erwähnte Haltevorrichtung 40 beibehalten wird, kann nun der Operateur die Bohrhilfe 50 ausrichten, indem er diese in gewünschter Weise um den Bolzen 54 dreht.

Dadurch, dass in dem Block 52 der Bohrhilfe 50 zwei Führungskanäle 64 und 66 ausgespart sind und diese auf unterschiedlichen Höhenniveaus und aufeinander zu gerichtet verlaufen, können nun zwei Bohrkanäle 82 und 84 längs der entsprechenden Führungsachsen 65 und 67 ausgebildet werden, durch die dann entsprechende Fixierschrauben oder Fixierdrähte hindurch geschoben werden können. Somit können in einem Vorgang zwei solche Bohrkanäle 82 und 84 bewerkstelligt werden, die nach Abziehen des Bohrwerkzeuges sehr gut zugänglich sind, um die Fixierschrauben oder Fixierdrähte zu setzen.

In Fig. 6 ist dargestellt, dass sich die Bohrhilfe 50 etwa in der in Fig. 3A dargestellten "Mittenstellung" befindet.

Durch entsprechendes Verschwenken der Bohrhilfe 50 um den Bolzen 54 können die Bohrkanäle 82 und 84 auch an anderen Stellen gesetzt werden, beispielsweise ein Kanal in der Darstellung von Fig. 6 weiter oben, ein anderer weiter unten - je nachdem, wie dies zur Fixierung des abgebrochenen Knochenstückes 79 am günstigsten ist.

Dies zeigt die einfache und variable Handhabung der Repositionszange 10 bei einem Eingriff.

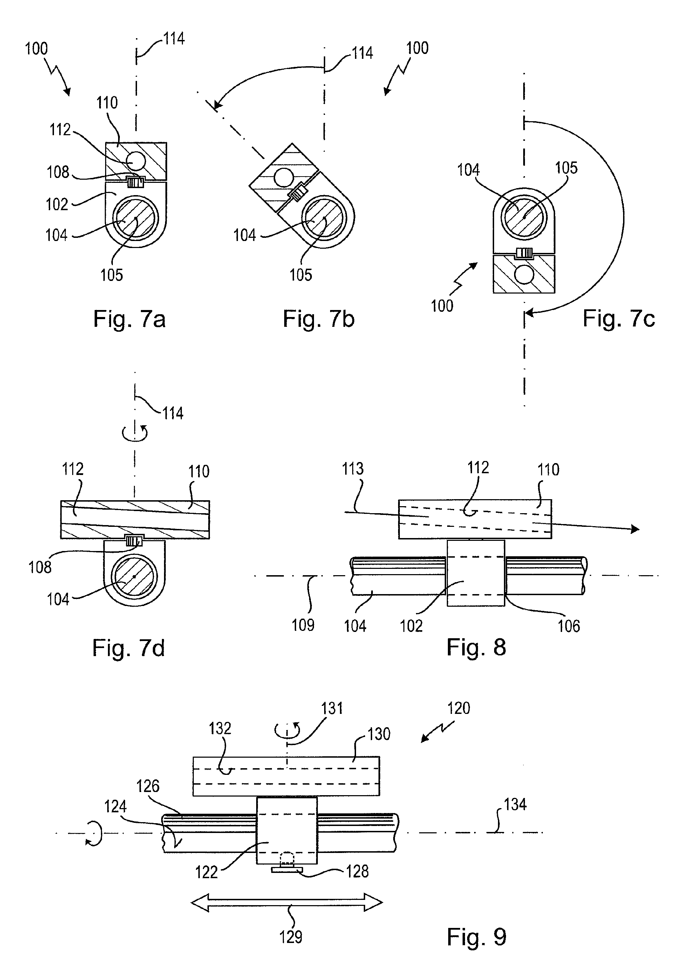

In den Fig. 7a bis 8 ist ein zweites Ausführungsbeispiel einer Repositionszange dargestellt, die, was den eigentlichen Zangenkörper betrifft, gleich aufgebaut ist wie die in Fig. 1 dargestellte Repositionszange 10.

Auch bei diesem Ausführungsbeispiel ist eine in ihrer Gesamtheit mit der Bezugsziffer 100 bezeichnete Bohrhilfe vorgesehen, die an einer Branche 104 angebracht ist, wobei die Branche 104 der Branche 22 in Fig. 1 entspricht.

Aus dem Querschnitt, der quer zu einer Mittellängsachse 105 der Branche 104 verläuft, ist ersichtlich, dass die Bohrhilfe 100 eine Hülse 102 aufweist, die den Körper der Branche 104 hier vollständig umgreift.

Die Hülse 102 sitzt in einer umlaufenden Nut 106 an der Außenseite der Branche 104. Am in der Darstellung von Fig. 7a oberen oder der Branche 104 abgewandten Ende ist ein Drehzapfen 108 eingelassen, auf dem drehbeweglich ein Führungsblock 110 der Bohrhilfe 100 montiert ist.

Die Mittellängsachse des Drehzapfens 108 bildet eine Drehachse 114, um die der Führungsblock 110 drehbar ist. Aus der Schnittdarstellung von Fig. 7a ist auch ersichtlich, dass im Führungsblock 110 ein Führungskanal 112 ausgespart ist. Aus der Folge von Fig. 7a, 7b und 7c ist ersichtlich, dass die gesamte Bohrhilfe 100 beliebig um die Mittellängsachse 105 der Branche 104 drehbar ist.

Vom Übergang von Fig. 7a zu Fig. 7b ist ersichtlich, dass die Bohrhilfe 100 etwa um 30° entgegen dem Uhrzeigersinn verschwenkt worden ist. Aus der Darstellung von Fig. 7c ist ersichtlich, dass die Bohrhilfe 100 etwa um 180° um die Mittellängsachse 105 verschwenkt worden ist. Diese Verschwenkung oder Drehbewegung wird exakt dadurch durchgeführt, dass die Hülse 102 in einer in die Außenseite der Branche 104 eingeschnittene Nut 160 eingesetzt und von dieser geführt ist. Diese Verschwenkbarkeit um die Längsachse 105 der Branche 104 kann entsprechend schwergängig gemacht werden, so dass die Bohrhilfe 100 in der jeweiligen Drehstellung verbleibt. Es kann auch eine Feststellvorrichtung, zum Beispiel eine radiale Feststellschraube, vorgesehen sein.

In den Fig. 7a bis 7c ist dargestellt, dass sich der Führungskanal 112 in Richtung der Mittellängsachse 105 der Branche 104 erstreckt.

Wie zuvor erwähnt, ist aber der Führungsblock 110 um die Drehachse 114 drehbar.

In Fig. 7d ist dargestellt, wie der Führungsblock 110 um 90° um die Drehachse 114 gegenüber der Ausrichtung von Fig. 7a verdreht ist.

Aus den Darstellungen von Fig. 7d und Fig. 8 ist ersichtlich, dass die Führungsachse 113 des Führungskanals 112 geneigt zur Zangenebene 109 verläuft.

Je nachdem, von welcher Seite aus man nun das Bohrwerkzeug durch den Führungskanal 112 einschiebt, oder in welcher Drehrichtung er sich befindet, kann nun eine Bohrung bewerkstelligt werden, die entweder auf die Zangenebene 109 zu geneigt oder von dieser weg gerichtet ist.

Dies zeigt wiederum die Variabilität für die Handhabungsperson zur Setzung einer geeigneten Bohrung im Knochen.

Die geneigte Führung der Führungsachse 113 ist nur ein dargestelltes Ausführungsbeispiel; der Führungskanal 112 kann sich auch parallel zur Zangenebene 109 erstrecken.

In Fig. 9 ist ein drittes Ausführungsbeispiel einer Repositionszange dargestellt, wobei auch hier wieder die Repositionszange als solche gleich ausgebildet ist wie die in Fig. 1 dargestellte Repositionszange 10.

Im Unterschied zu dem in den Fig. 7 und 8 dargestellten zweiten Ausführungsbeispiel ist beim dritten Ausführungsbeispiel der Bohrhilfe 120 die Hülse 122 so ausgebildet, dass sie die Außenfläche 124 der Branche 126, die wiederum der Branche 22 des Ausführungsbeispiels von Fig. 1 entspricht, vollständig umgreift und an dieser anliegt.

In der Branche 126 ist eine Feststellvorrichtung 128, beispielsweise in Form einer sich radial erstreckenden Feststellschraube, vorhanden, um eine bestimmte Drehstellung der Hülse 122 um die Längsachse 132 der Branche 126 zu fixieren. Die Mittellängsachse der Branche 126 liegt in der Zangenebene 134 der Repositionszange.

Auch hier ist an der Hülse ein Führungsblock 130 über einen Drehzapfen drehbar montiert, so dass der Führungsblock 130 um eine senkrecht zur Zangenebene 134 stehende Drehachse 131 verdreht werden kann.

Durch den Führungsblock 130 erstreckt sich ein durchgehender Führungskanal 132, der nicht geneigt ist, das heißt, dessen Mittellängsachse verläuft parallel zur Zangenebene 134.

Bei diesem Ausführungsbeispiel ist wieder der Führungsblock 130 mit dem Führungskanal 132 um eine Drehachse 131 um die Hülse 122 drehbar.

Außerdem ist die Hülse 122 selbst und somit die komplette Bohrhilfe 120 um die Längsachse der Branche 126 drehbar, wie bereits zuvor beschrieben wurde.

Zusätzlich besteht nun noch der dritte Freiheitsgrad, dass die gesamte Bohrhilfe 120 durch Lösen der Feststellvorrichtung auch noch längs der Branche 136 verschoben werden kann, wie das durch den Doppelpfeil 129 angedeutet ist.

In dieser Ausführung stehen dem Operateur nun zahlreiche Freiheitsgrade zur Verfügung, damit die Bohrhilfe in eine für den jeweiligen Fall maximal günstige Ausrichtung gebracht werden kann.

Es ist selbstverständlich, dass bei den im zweiten und dritten Ausführungsbeispiel gezeigten Bohrhilfen auch mehrere Bohrkanäle ausgebildet sein können, wie sie im Zusammenhang mit Fig. 1 beschrieben sind.

## Patentansprüche

1. Repositionszange zum Halten von zwei zu verbindenden Knochenteilen (78, 79), mit zwei Zangenhälften (12, 14), die über ein Gelenk (16) scherenartig verbunden und in einer Zangenebene (18, 134) verschwenkbar sind, wobei jede Zangenhälfte (12, 14) distalseitig des Gelenkes (16) eine seitlich ausgebogene Branche (20, 22; 104, 126) und proximalseitig ein Griffteil (20, 22) aufweist, und wobei an einer Branche (22, 104, 126) eine Bohrhilfe (50, 100, 120) bewegbar angebracht ist, die eine Führungsachse (65, 67, 113) für ein Bohrwerkzeug aufweist, **dadurch gekennzeichnet, dass** die Bohrhilfe (50, 100, 120) zumindest um eine Drehachse (58, 114, 131) drehbar ist, die senkrecht zur Zangenebene (109, 134) steht, und wobei sich eine Führungsachse (65, 67, 113) in einer Drehstellung der Bohrhilfe (50, 100, 120) längs der Branche (20, 22; 104, 126) erstreckt.

2. Repositionszange nach Anspruch 1, **dadurch gekennzeichnet, dass** die Bohrhilfe (50, 100, 120) als Block (52, 110, 130) ausgebildet ist, durch den sich zumindest ein Führungskanal (64, 66; 112, 132) hindurch erstreckt.

3. Repositionszange nach Anspruch 2, **dadurch gekennzeichnet, dass** im Block (52) zumindest zwei Führungskanäle (64, 66) ausgebildet sind.

4. Repositionszange nach Anspruch 2 oder 3, **dadurch gekennzeichnet, dass** Führungsachsen (65, 67) der Führungskanäle (64, 66) auf verschiedenen Höhenniveaus (72, 74) und/oder nicht-parallel zueinander verlaufen, aber dennoch auf einen Bereich zwischen den beiden Branchen (20, 22) ausrichtbar sind.

5. Repositionszange nach einem der Ansprüche 1 bis 4, **dadurch gekennzeichnet, dass** die Bohrhilfe (50) über einen sich in der Drehachse (58) erstreckenden Bolzen (54) mit der Branche (22) verbunden ist.

6. Repositionszange nach einem der Ansprüche 1 bis 5, **dadurch gekennzeichnet, dass** das Ausmaß der Drehbarkeit der Bohrhilfe (50) um die Drehachse (58) begrenzt ist.

7. Repositionszange nach Anspruch 6, **dadurch gekennzeichnet, dass** von der Bohrhilfe (50) beidseits der Branche (22) Anschläge (60, 62) vorstehen, die ein Drehen um die Drehachse (58) in entgegengesetzten Drehrichtungen jeweils begrenzen.

8. Repositionszange nach einem der Ansprüche 1 bis 7, **dadurch gekennzeichnet, dass** die Bohrhilfe (100, 120) zusätzlich um eine Längsachse (105, 134) der Branche (104, 126), an der diese angebracht ist, drehbar ausgebildet ist.

9. Repositionszange nach einem der Ansprüche 1 bis 8, **dadurch gekennzeichnet, dass** die Bohrhilfe (120) zusätzlich längs der Branche (126), an der diese angebracht ist, verschiebbar ausgebildet ist.

10. Repositionszange nach einem der Ansprüche 1 bis 9, **dadurch gekennzeichnet, dass** die beiden Branchen (20, 22) der Zangenhälften (12, 14) distal als nadelartige Dorne (28, 30) ausgebildet sind.

11. Repositionszange nach Anspruch 10, **dadurch gekennzeichnet, dass** die Branchen (20, 22), von einer Mittellängsachse (17) der Repositionszange (10) aus gesehen, seitlich ausgebogene Abschnitte (24, 26) aufweisen, deren nadelartigen Dorne (28, 30) bei geschlossener Repositionszange (10) aufeinander zu gerichtet sind.

12. Repositionszange nach Anspruch 10 oder 11, **dadurch gekennzeichnet, dass** die Bohrhilfe (50) in der Nähe des nadelartigen Dornes (30) angeordnet ist.

13. Repositionszange nach einem der Ansprüche 1 bis 12, **dadurch gekennzeichnet, dass** eine sich zwischen den Griffteilen (32 und 34) erstreckende Haltevorrichtung (40) vorhanden ist, über die die Griffteile (32 und 34) in bestimmten Abständen haltbar sind.
